# EUROPÄISCHE PATENTANMELDUNG

(11) **EP 2 204 151 A2**
(43) Veröffentlichungstag der Anmeldung: **07.07.2010**
(21) Anmeldenummer: 10001059.4
(22) Anmeldetag: 03.03.2006
(51) Int. Cl.: A61F 9/008, B23K 26/00, H01S 3/00, B23K 26/06

(54) **Vorrichtung und Verfahren zur photodisruptiven Laserbearbeitung eines toten Materials**

(62) Teilanmeldung aus: 06004388.2
(71) Anmelder: WaveLight AG, 91058 Erlangen (DE); Fraunhofer-Gesellschaft zur Förderung der angewandten Forschung e.V., 80686 München (DE)
(72) Erfinder: Vogler, Klaus, Dr., 90542 Eckental (DE); Tünnermann, Andreas, Prof., 99425 Weimar (DE)
(74) Vertreter: Katérle, Axel

(57) **Zusammenfassung**

Ein Verfahren zur photodisruptiven Laserbearbeitung eines toten Materials gibt einen Zug (18') von Laserstrahlungspulsen mit einer Pulsdauer im Femtosekundenbereich auf das Material ab. Der Pulszug umfasst eine Vielzahl aufeinanderfolgender Pulsgruppen, wobei jede Pulsgruppe mindestens zwei Laserstrahlungspulse (20', 22') umfasst. Die Pulse einer Pulsgruppe werden im wesentlichen auf denselben Bearbeitungsort des Materials gerichtet, die Pulse aufeinanderfolgender Gruppen jedoch auf im wesentlichen verschiedene Bearbeitungsorte des Materials. Erfindungsgemäß liegt der zeitliche Abstand zweier aufeinanderfolgender Laserstrahlungspulse einer Pulsgruppe im Nanosekundenbereich. Insbesondere wird die Intensität bzw. Energie der Pulse innerhalb einer Pulsgruppe abgestuft, nämlich so, dass ein vorrauslaufender Vorpuls (20') wesentlich geringere Intensität bzw. Energie als ein nachlaufender Hauptpuls (22') hat.

## Beschreibung

Die Erfindung betrifft eine Vorrichtung und ein Verfahren zur photodisruptiven Laserbearbeitung eines toten Materials.

Grundsätzlich ist die Erfindung nicht auf bestimmte Materialien beschränkt. Es ist eine Anwendung der Erfindung sowohl bei anorganischen als auch bei organischen Materialien denkbar. Die nachfolgenden Ausführungen erfolgen mit besonderem Blick auf die laserchirurgische Bearbeitung der Kornea mit dem Ziel der Behebung von Fehlsichtigkeiten, wobei zu betonen ist, dass die Erfindung zur Anwendung bei toter Materie gedacht ist.

Der Einsatz von Femtosekundenlasern zur Materialbearbeitung ist bekannt. Ein Femtosekundenlaser stellt gepulste Laserstrahlung mit ultrakurzen Pulsdauern im Femtosekundenbereich bereit. Der Begriff Femtosekundenbereich soll im Rahmen der Erfindung so verstanden werden, dass er insbesondere auch Pulslängen im hohen dreistelligen Femtosekundenbereich sogar bis hin zu einstelligen Pikosekunden umfasst. Was den Begriff Pulsdauer bzw. Pulslänge anbelangt, so bezieht sich dieser auf einen statistischen Mittelwert, denn es versteht sich, dass die einzelnen Pulse des Lasers in der Regel nicht alle exakt gleich lang sind und exakt gleichen Intensitätsverlauf haben. Als Pulsdauer eines Laserstrahlungspulses kann beispielsweise seine Halbwertslänge angegeben werden.

Marktgängige Femtosekundenlaser für die Materialbearbeitung umfassen einen Laseroszillator, der Femtosekundenpulse mit einer Wiederholrate bzw. Pulsfolgefrequenz im MHz-Bereich, beispielsweise in der Größenordnung von 1 MHz bis 100 MHz, und mit einer vergleichsweise niedrigen Pulsenergie im niederen nJ-Bereich, beispielsweise zwischen 0,1 und 10 nJ, erzeugen kann. Die Pulswiederholrate im MHz-Bereich entspricht einem Zeitabstand zwischen aufeinanderfolgenden Pulsen des Laseroszillators im Nanosekundenbereich.

Zur Verstärkung der in der Regel relativ energieschwachen Pulse üblicher fs-Laseroszillatoren ist es gemäß einer in der Fachwelt als *chirped pulse amplification* (CPA) bezeichneten Vorgehensweise bekannt, die Pulse ggf. nach einer Vorverstärkung zunächst zeitlich zu strecken, bevor sie bei vergleichsweise niedrigem Intensitätsniveau verstärkt werden. Anschließend werden die Pulse wieder zeitlich komprimiert. Auf diese Weise können sehr energiereiche und dennoch extrem kurze Laserstrahlungspulse erzeugt werden. Die Energie der so erzeugten Pulse liegt beispielsweise im µJ- oder sogar im mJ-Bereich. Sie eignen sich hervorragend zur präzisen, nichtthermischen Mikrobearbeitung von Materialien, weswegen Femtosekundenlaser auch für Anwendungen in der Medizin, insbesondere in der Ophthalmologie, eingesetzt werden.

Beispielsweise ist es bei verschiedenen Verfahren der Augenchirurgie erforderlich, Gewebestrukturen im oder am Auge zu trennen. Bei der refraktiven Augenchirurgie beispielsweise, die durch Veränderung der Brechungseigenschaften der Kornea Fehlsichtigkeiten zu beheben versucht, sind bei einigen Verfahren Schnitte in die Kornea einzubringen, so etwa bei der LASIK (Laser Keratomileusis), wo aus dem Hornhautepithel ein an einem Teil seines Rands noch mit der Hornhaut zusammenhängendes Deckelscheibchen (bekannt als *Flap*) herausgetrennt wird, das zur Seite geklappt werden kann, um so die darunter liegenden Hornhautbereiche freizulegen. Bei einer LASIK-Variante erfolgt nach Wegklappen des Flaps durch Einstrahlung gepulster Laserstrahlung im niederen UV-Wellenlängenbereich eine Photoablation der freigelegten Bereiche des Stromas nach Maßgabe eines für den jeweiligen Patienten angefertigten Ablationsprofils. Bei einer anderen LASIK-Variante wird zunächst innerhalb der Hornhaut ein linsenartiges Scheibchen mittels eingestrahlter fs-Pulse freigeschnitten, bevor der Flap erzeugt wird. Dieses Linsenscheibchen entspricht dem aus der Kornea für die Behebung der Fehlsichtigkeit zu entfernenden Materialvolumen. Nach Wegklappen des Flaps kann dann das freigeschnittene Linsenscheibchen herausgenommen und der Flap wieder zurückgeklappt werden. Der Einsatz von Femtosekundenlasern bei der refraktiven Augenchirurgie wird im übrigen nicht nur für die Anbringung von Schnitten erwogen, sondern auch für eine mehrschichtige intrastromale Materialverdampfung, die keine kornealen Einschnitte mehr verlangt.

Zum Stand bei der Anwendung von Femtosekundenlasern in der refraktiven Augenchirurgie wird beispielhaft auf die folgenden beiden Publikationen verwiesen:
Tibor Juhasz et al.: "Corneal Refractive Surgery with Femtosecond Lasers", IEEE Journal of Selected Topics in Quantum Electronics, Vol. 5, No. 4, July/August 1999, pp. 902-910
C L. Arnold et al.: "Streak formation as side effect of optical breakdöwn during processing the bulk of transparent Kerr media with ultra-short laser pulses", Applied Physics B 80, 2005, pp. 247-253

Zumindest bei der refraktiven Femtosekunden-Augenchirurgie ist es bisher üblich, die gewünschte Gewebetrennung oder den gewünschten Gewebeabtrag mit einem Zug von sich in gleichen Zeitabständen wiederholenden fs-Einzelpulsen zu erzwingen, wobei die Pulsrepetitionsrate des in das Auge eingestrahlten Pulszugs je nach Leistungsfähigkeit des Hauptverstärkers des Femtosekundenlasers (oftmals wird hierbei ein regenerativer Verstärker eingesetzt) typischerweise zwischen einigen wenigen kHz und einigen wenigen hundert kHz liegt. Der Laserstrahl wird dabei so über den zu bearbeitenden Gewebebereich geführt, dass zwei aufeinanderfolgende Einzelpulse nicht auf dieselbe Stelle des Gewebes treffen, sondern auf benachbarte, wenngleich unter Umständen etwas überlappende Stellen.

DE 101 25 206 B4 schlägt für die Mikrostrukturierung von Materialien wie Quarzglas und Graphit mittels fs-Laserpulsen vor, statt Einzelpulsen Doppelpulse zu verwenden, die sich aus einem energieschwächeren Vorpuls und einem anschließenden energiestärkeren Hauptpuls zusammensetzen. Die Energie des Vorpulses und des Hauptpulses soll jeweils unter der Mikrostrukturierungsschwelle des zu bearbeitenden Materials liegen. Nur die Energie beider Teilpulse zusammen soll über dieser Schwelle liegen. Durch den Vorpuls sollen Primärprozesse in dem Material angeregt werden, die bei Eintreffen des Hauptpulses noch bemerkbar sein sollen. Dadurch könne das Material durch den Hauptpuls besser bearbeitet werden, ohne Risse und Spannungen im Material hervorzurufen.

Damit eine durch den Vorpuls in dem Material bewirkte Änderung bei Eintreffen des Hauptpulses noch bemerkbar ist, müsse der zeitliche Abstand des Hauptpulses vom Vorpuls auf einer Sub-Pikosekunden- oder einer Pikosekunden-Zeitskala liegen. Konkret wird für Quarzglas ein Zeitabstand von 0,6 ps und für Graphit ein Zeitabstand von 2 ps angegeben. Es ist nichts in dem Dokument darüber erwähnt, wie die Doppelpulse erzeugt werden, insbesondere nichts darüber, wie der äußerst kurze Zeitabstand zwischen Vor- und Hauptpuls stabil und zuverlässig erreicht werden kann.

Aufgabe der Erfindung ist es, einen Weg aufzuzeigen, wie bei der photodisruptiven Laserbearbeitung eines toten Materials hochwertige Ergebnisse erzielt werden können, bei denen unerwünschte Materialänderungen außerhalb der zu bearbeitenden Materialbereiche sehr gut vermieden werden können. Dabei sollen diese hochwertigen Ergebnisse zuverlässig erzielbar sein, d.h. mit relativ hoher Gleichmäßigkeit reproduzierbar sein.

Bei der Lösung dieser Aufgabe geht die Erfindung aus von einem Verfahren zur photödisruptiven Laserbearbeitung eines toten Materials, wobei bei diesem Verfahren ein Zug von Laserstrahlungspulsen mit einer Pulsdauer im Femtosekundenbereich in Richtung auf das Material abgegeben wird, wobei der Pulszug eine Vielzahl aufeinanderfolgender Pulsgruppen umfasst, wobei jede Pulsgruppe mindestens zwei Laserstrahlungspulse umfasst, wobei bei dem Verfahren die Laserstrahlungspulse einer Pulsgruppe im wesentlichen auf denselben Bearbeitungsort des Materials gerichtet werden, jedoch die Laserstrahluhgspulse aufeinanderfolgender Putsgruppen auf im wesentlichen verschiedene Bearbeitungsorte des Materials gerichtet werden.

Erfindungsgemäß ist dabei vorgesehen, dass der zeitliche Abstand zweier aufeinanderfolgender Laserstrahlungspulse einer Pulsgruppe im Nanosekundenbereich, insbesondere im niederen Nanosekundenbereich liegt. Untersuchungen haben gezeigt, dass insbesondere in transparenten biologischen Materialien und hier wiederum insbesondere in Hornhautgewebe mit einem energetisch oder/und intensitätsmäßig geeignet abgestimmten fs-Vorpuls vergleichsweise lang anhaltend solche Bedingungen geschaffen werden können; die eine hochwirksame Einkopplung eines anschließenden fs-Hauptpulses in das Material an der gewünschten Stelle gestatten. Mit lang anhaltend ist hier ein Zeitraum gemeint, der wesentlich länger als der in DE 101 25 206 B4 gelehrte Zeitabstand von höchstens einstelligen Pikosekunden zwischen Vor- und Hauptpuls ist.

Tatsächlich hat sich beispielsweise gezeigt, dass im Hornhautgewebe mit einem geeigneten Vorpuls Mikroveränderungen erzeugbar sind, die bis zu 10 ns oder sogar darüber hinaus bestehen können. Dementsprechend "weit" können die Pulse einer Pulsgruppe in dem beim erfindungsgemäßen Verfahren abgegebenen Pulszug auseinander liegen. Der Vorteil daran ist, dass aufeinanderfolgender Pulse mit einem Zeitabstand im Nanosekundenbereich mit handelsüblichen Bauteilen der Optik und Optoelektronik leichter stabil und mit gleichmäßigen Eigenschaften erzeubar sind als Pulse mit einem Abstand von höchstens einigen Pikosekunden. Dies gilt sowohl für die Pulse selbst als auch für den Zeitabstand zwischen den Pulsen. Die bessere Reproduzierbarkeit führt dann zwangsläufig zu besseren Ergebnissen der Materialbearbeitung. Der Begriff Nanosekundenbereich soll im Rahmen der Erfindung keinesfalls so eng ausgelegt werden, dass er dreistellige Pikosekunden von vorneherein ausschließt. Im Gegenteil sollen insbesondere hohe dreistellige Pikosekundenabstände zwischen aufeinanderfolgenden Pulsen einer Pulsgruppe als ebenfalls im Umfang der Erfindung liegend angesehen werden. Die Mikroveränderungen, die im bearbeiteten Material durch einen Vorpuls bewirkt werden können, können beispielsweise in Form von Mikroschädigungen des Materials oder/und in Form einer erhöhten Menge an freien Elektronen vorliegen. Solche durch den Vorpuls freigesetzten Elek-tronen können bei Eintreffen eines anschließenden Hauptpulses zu einem rascheren oder/und effektiveren Lawinenprozess führen, der letztlich die Photodisruption bewirkt.

Der zeitliche Abstand zweier aufeinanderfolgender Laserstrahlungspulse einer Pulsgruppe kann gemäß einer bevorzugten Ausführungsform weniger als 100 ns, vorzugsweise weniger als 50 ns und höchstvorzugsweise weniger als 20 ns betragen. Beispielsweise kann dieser Zeitabstand im einstelligen Nanosekundenbereich bis hin zu höchstens etwa 10 ns liegen.

Bevorzugt besitzt von zwei aufeinanderfolgenden Laserstrahlungspulsen einer Pulsgruppe ein vorlaufender Puls geringere Energie oder/und geringere maximale Intensität als der nachfolgende Puls. Dabei ist die Energie oder/und die maximale Intensität des vorlaufenden Pulses vorzugsweise höchstens die Hälfte, besser höchstens ein Viertel und noch besser höchstens ein Zehntel der Energie bzw. der maximalen Intensität des nachfolgenden Pulses. Beispielsweise kann die Energie bzw. maximale Intensität des vorlaufenden Pulses näherungsweise ein Zwanzigstel derjenigen des folgenden Pulses betragen.

Jede Pulsgruppe kann gemäß einer Ausführungsform insgesamt zwei Laserstrahlungspulse umfassen. Es ist freilich im Rahmen der Erfindung nicht ausgeschlossen, dass die Pulsgruppen jeweils drei oder mehr Laserstrahlungspulse umfassen. Der erfindungsgemäße nanoskalige Zeitabstand zwischen aufeinanderfolgenden Pulsen einer Pulsgruppe kann dabei nur für einen Teil der benachbarten Pulspaare einer Pulsgruppe oder für alle benachbarten Pulspaare der Pulsgruppe vorgeschrieben sein. Enthält eine Pulsgruppe drei oder mehr Pulse, so können alle Pulse der Gruppe unterschiedliche Energie oder/und maximale Intensität haben. Insbesondere kann die Energie oder/und maximale Intensität vom ersten bis zum letzten Puls der Gruppe zunehmend größer werden. Alternativ ist es vorstellbar, dass ein Teil der Pulse einer Gruppe, insbesondere am Ende der Gruppe, näherungsweise gleiche Energie oder/und maximale Intensität hat.

Eine das Verfahren ausführende Laserbearbeitungsvorrichtung kann eine Strahlablenkeinrichtung (sogenannter *Scanner*) enthalten, mittels der der Laserstrahl über einen Bereich des zu bearbeitenden Materials hinwegbewegt werden kann. Generell wird dabei die Aufgabe bestehen, die Ablenkgeschwindigkeit des Scanners und die Zeitabstände zwischen den Pulsen einer Pulsgruppe und zwischen den Gruppen so aufeinander abzustimmen, dass die Pulse einer Gruppe im wesentlichen auf denselben Ort einstrahlen, aufeinanderfolgende Pulsgruppen jedoch im wesentlichen auf verschiedene Orte einstrahlen. Diesbezüglich wird empfohlen, den zeitlichen Abstand zweier aufeinanderfolgender Pulsgruppen wenigstens eine Größenordnung, vorzugsweise mehrere Größenordnungen größer als den zeitlichen Abstand aufeinanderfolgender Laserstrahlungspulse einer Pulsgruppe einzustellen.

Wie oben erwähnt, gibt es marktgängige Femtosekundenlaser, deren Laseroszillator Laserstrahlungspulse mit einer Wiederholrate im MHz-Bereich erzeugen kann, also mit Zeitabständen im Bereich von Nanosekunden. Es sind auch bereits Femtosekundenlaser bekannt, die mittels eines elektro-optischen Schalters, etwa einer Pockels-Zelle, einzelne Pulse aus der von dem Oszillator erzeugten kontinuierlichen Pulsfolge selektieren können und nur die selektierten Pulse, in der Regel nach Verstärkung, als Bearbeitungsstrahlung abgeben. Mit einem solchen elektro-optischen Schalter ergibt sich die vorteilhafte Möglichkeit, für jeden Laserstrahlungspuls des abgegebenen Pulszugs mit Hilfe des Schalters jeweils einen Puls aus den von dem Laseroszillator erzeugten Pulsen zu selektieren. Dies bedeutet, dass nicht aufwendig und schlecht kontrollierbar aus einem Einzelpuls ein Doppelpuls erzeugt werden muss, um die Pulse einer Pulsgruppe zu bilden. Stattdessen können alle Pulse einer Gruppe und somit des gesamten Zugs durch Einzelselektion aus der Pulsfolge des Laseroszillators gewonnen werden, d.h. jeder abgegebene Behandlungspuls entspricht jeweils einem Laserpuls des Oszillators. Ist die Pulswiederholrate des verwendeten Laseroszillators so hoch, dass der Zeitabstand aufeinanderfolgender Pulse des Oszillators im Bereich von Nanosekunden liegt, insbesondere im niederen Nanosekundenbereich, haben zwei aufeinanderfolgende Pulse des Oszillators automatisch den erfindungsgemäß für zwei aufeinanderfolgende Pulse einer Pulsgruppe geforderten Zeitabstand. Der Gedanke, alle Pulse einer Pulsgruppe durch Einzelselektion aus der Pulsfolge eines geeignet hochfrequenten fs-Laseroszillators zu gewinnen, wird als selbständig schutzfähig angesehen.

Zur Erzeugung von unterschiedlich intensitätsstarken Pulsen in dem Zug können Intensitätsmodulationsmittel vorgesehen sein, welche die Einstellung einer gewünschten Intensität jedes selektierten Pulses gestatten. Besonders vorteilhaft dabei ist, dass als Intensitätsmodulator ein auch für die Pulsselektion nutzbarer elektro-optischer Schalter verwendet werden kann, etwa die erwähnte Pockels-Zelle. Je nach gewünschter Pulsintensität wird der Schalter dann mehr oder weniger stark geöffnet. Es versteht sich, dass für Pulsselektion und Pulsformung bei Bedarf auch gesonderte Komponenten vorgesehen sein können.

Nachfolgend wird die Erfindung anhand der beigefügten Zeichnungen weiter erläutert. Es stellen dar:
Figur 1 schematisch ein Ausführungsbeispiel einer Vorrichtung zur refraktiven laserchirurgischen Bearbeitung des Auges;
Figur 2 schematisch eine Anordnung zur Einzelpulsselektion aus einer regelmäßigen Folge von Laserstrahlungspulsen und
Figur 3 eine Darstellung zur Erläuterung des Prinzips der Einzelpulsselektion und Pulsformung mittels eines elektro-optischen Schalters.

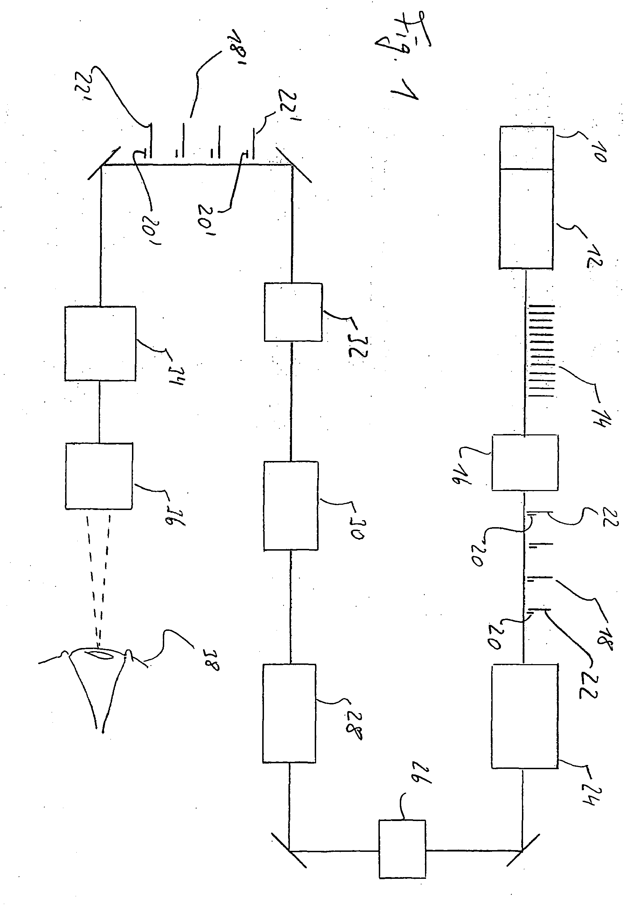

Es wird zunächst auf Figur 1 verwiesen. Die in dieser Figur schematisch dargestellte Laserbearbeitungsvorrichtung umfasst einen von einem Pumplasermodul 10 gepumpten Laseroszillator 12, der eine Folge von Laserstrahlungspulsen erzeugt, die in regelmäßigen Abständen aufeinanderfolgen. Die Pulsfolge ist schematisch bei 14 angedeutet. Die Zeitdauer der einzelnen Pulse liegt im Femtosekundenbereich, beispielsweise bei etwa 200fs. Die Pulswiederholrate des Laseroszillators 12 liegt im MHz-Bereich, insbesondere im dreistelligen MHz-Bereich, so dass sich ein Abstand der Pulse der Folge 14 von etwa 10ns oder sogar darunter ergibt. Fs-Laseroszillatoren mit Pulsrepetitionsraten von 100 MHz, 200 MHz oder sogar mehr sind als solche handelsüblich erhältlich. Die Wellenlänge der erzeugten Laserstrahlung liegt beispielsweise im niederen infraroten Bereich, etwa zwischen 1000 und 1100 µm. Andere Wellenlängen sind im Rahmen der Erfindung jedoch gleichermaßen möglich, insbesondere auch Wellenlängen im UV-Bereich.

Aus der von dem Laseroszillator 12 erzeugten Pulsfolge 14 selektiert ein Pulsselektor 16 durch Einzelpulsselektion einen bei 18 schematisch angedeuteten Pulszug, der sich aus aufeinanderfolgenden Pulsgruppen von je einem energie- bzw. intensitätsschwächeren vorlaufenden Puls 20 und einem energie- bzw. intensitätsstärkeren nachlaufenden Puls 22 zusammensetzt. Jeder der Pulse 20, 22 wird durch Einzelselektion eines jeweiligen Pulses der Folge 14 selektiert. Der zeitliche Abstand der Pulse 20, 22 einer Pulsgruppe entspricht dem Zeitabstand zwischen den Pulsen der Folge 14, d.h. die Pulse 20, 22 einer Pulsgruppe werden durch Selektion zweier unmittelbar aufeinanderfolgender Pulse der Folge 14 gebildet. Bei den vorstehend erwähnten beispielhaften Pulswiederholraten des Laseroszillators 12 beträgt somit der Zeitabstand zwischen den Pulsen 20, 22 einer Pulsgruppe etwa 10ns oder weniger. Es versteht sich, dass die Pulse einer Pulsgruppe nicht grundsätzlich aus unmittelbar aufeinanderfolgenden Pulsen der Folge 14 hervorgehen müssen. Vielmehr ist es auch vorstellbar, dass der Pulsselektor 16 für eine Pulsgruppe Pulse aus der Folge 14 selektiert, die durch mindestens einen Zwischenpuls getrennt sind.

Der Zeitabstand zwischen den Pulsgruppen des Pulszugs 18 beträgt dagegen ein Vielfaches des Zeitabstandes zwischen den Pulsen 20, 22 einer Gruppe. Beispielsweise kann der Zeitabstand zwischen den Pulsgruppen im Mikrosekundenbereich liegen.

Es versteht sich, dass bei einer abgewandelten Ausführungsform jede Pulsgruppe mehr als zwei Einzelpulse enthalten kann, beispielsweise drei.

Der Pulsselektor 16 ist als elektro-optischer Schalter ausgebildet, welcher aus der erzeugten Pulsfolge 14 einzelne Pulse mit einstellbarer Intensität selektieren kann. Je nachdem, wie stark der elektro-optische Schalter "geöffnet" ist, sind die selektierten Pulse intensitätsmäßig mehr oder weniger abgeschwächt. Durch entsprechende Ansteuerung des Pulsselektors 16 ist somit der in Figur 1 dargestellte Pulszug 18 realisierbar. Als elektro-optischen Schalter kann der Pulsselektor 16 beispielsweise eine Pockels-Zelle enthalten. Bei einer Pockels-Zelle kann über das Niveau der angelegten Spannung die Stärke der Abschwächung der selektierten Pulse (d.h. im wesentlichen keine Abschwächung bei den Pulsen 22, jedoch eine signifikante Abschwächung bei den Pulsen 20) bestimmt werden.

Die selektierten Pulse 20, 22 können anschließend in einem optionalen Vorverstärker 24 vorverstärkt werden, bevor sie mittels *chirped pulse amplification* eine Hauptverstärkung erfahren. Bei dieser CPA-Verstärkung werden die Pulse 20, 22 zeitlich reversibel gedehnt und auf ein moderates oder endgültiges Energieniveau verstärkt. Die Pulsdehnung, die in einem sogenannten Stretcher 26 erfolgt, wird mittels eines dispersiven Elements bewirkt, das die unterschiedlichen Laufzeiten verschiedener spektraler Pulsanteile beim Durchgang durch das dispersive Element zur Pulsverbreiterung nutzt. Durch die Pulsverbreiterung wird das Intensitätsniveau der Pulse herabgesetzt, so dass bei der Verstärkung in einem fs-Hauptverstärker 28 parasitäre nichtlineare Effekte vermieden werden können. Der Hauptverstärker 28 kann beispielsweise ein regenerativer Verstärker sein.

Die am Ausgang des Hauptverstärkers 28 bereitstehenden verstärkten Pulse können in einem optionalen Nachverstärker 30 nachverstärkt werden, bevor sie mittels eines Kompressors 32 wieder komprimiert werden. Der Kompressor 32 kompensiert durch Verwendung eines dispersiven Elements mit umgekehrten Laufzeiteffekten die Pulsdehnung des Stretchers 26 im Idealfall vollständig. Der Hauptverstärker 28 kann eine weitere Pulsselektionseinheit (in Figur 1 nicht gesondert bezeichnet) enthalten, die beispielsweise mit der sogenannten Cavity-Dump-Methode die verstärkten Pulse nochmals selektiert und hierdurch schärfer vom Untergrund abgrenzt. Der Nachverstärker 30 kann eine Teleskopanordnung zur Strahlaufweitung enthalten.

Am Ausgang des Kompressors 32 steht ein Pulszug 18' bereit, der dem Muster des Pulszugs 18 entspricht, dessen Pulse 20', 22' jedoch insgesamt energie- und intensitätsstärker als die Pulse 20, 22 des Pulszugs 18 sind. Das Energie- bzw. Intensitätsverhältnis zwischen den Pulsen 20' und den Pulsen 22' entspricht vorzugsweise im wesentlichen dem zwischen den Pulsen 20 und 22. Dieses Verhältnis kann beispielsweise im Bereich um den Wert 10 liegen, d.h. die Energie bzw. Intensität der Pulse 22' ist um etwa diesen Wert höher als die Energie bzw. Intensität der Pulse 20'. Auch die Pulslänge der Pulse 20', 22' entspricht vorzugsweise im wesentlichen derjenigen der Pulse 20, 22. Wegen in der Regel nicht erreichbarer vollständig idealer Kompensation der Dispersion des Stretchers 26 durch den Kompressor 32 kann es sein, dass die Pulse 20', 22' etwas längere Zeitdauer als die Pulse 20, 22 haben. In jedem Fall liegt jedoch die Pulsdauer der Pulse 20', 22' ebenfalls im Femtosekundenbereich, um die gewünschte nahezu athermische Materialbearbeitung bei minimaler lateraler Schädigung zu ermöglichen.

Die Pulse 20', 22' werden anschließend mittels einer Ablenkeinheit 34 und einer Fokussiereinheit 36 auf das zu bearbeitende Zielgebiet (hier die Kornea eines menschlichen Auges 38) gerichtet. Die Ablenkeinheit 34 bewirkt eine solche Ablenkung der auf die Kornea einfallenden Laserstrahlung, dass zur selben Pulsgruppe gehörende Pulse 20', 22' im wesentlichen auf denselben Ort auf oder innerhalb der Kornea einfallen, während die Pulse aufeinanderfolgender Pulsgruppen im wesentlichen auf benachbarte Ort einfallen. Der energieschwächere Vorpuls 20' einer Pulsgruppe bewirkt durch Multiphotonen-Ionisation die Ausbildung eines Mikroplasmas am Zielpunkt in der Kornea. Der darauffolgende energiestärkere Hauptpuls 22' kann effizient in dieses Plasma - oder allgemein: in das durch den Vorpuls modifizierte Materialvolumen - einkoppeln und eine wirksame Photodissektion des Stromagewebes ermöglichen. Wegen der bereits vorhandenen Mikroveränderungen des Hornhautmaterials transmittiert von dem Hauptpuls 22', wenn überhaupt, nur ein vergleichsweise geringer Anteil durch den Fokus hindurch und gelangt auf die Retina. Der von dem Vorpuls 20' bis zur Ausbildung der Mikromaterialveränderung durch den Fokus hindurchgehende Teil ist dagegen vergleichsweise gering, weswegen die Strahlenbelastung der Retina wesentlich gegenüber herkömmlichen Korneabearbeitungsverfahren mit Einzelpulseinstrahlung reduziert werden kann. Die Energie der Hauptpulse 22' kann beispielsweise im einstelligen µJ-Bereich bis hin zu 10µJ oder sogar darüber liegen. Die Energie der Vorpulse 20' beträgt dagegen - wie weiter oben schon angedeutet - beispielsweise nur etwa 1/10 dieser Energie.

Um eine rein beispielhafte, zahlenmäßige Abschätzung zu geben, sei angenommen, dass der Zeitabstand der auf das Zielgebiet eingestrahlten Pulse einer Pulsgruppe 10 ns beträgt, der Pulsgruppenabstand (also der Zeitabstand zweier aufeinanderfolgender Pulsgruppen) 5 µs beträgt und die Ablenkgeschwindigkeit des Scanners 1 m/s beträgt. Bei diesen Zahlenwerten treffen die aufeinanderfolgenden Pulsgruppen in einem Ortsabstand von 5 µm auf dem Zielgebiet ein, während der Ortsunterschied zweier aufeinanderfolgender Pulse einer Pulsgruppe nur 0,01 µm ist. Der Ortsabstand der Pulse einer Pulsgruppe ist damit um Größenordnungen kleiner als der Ortsabstand zweier aufeinanderfolgender Pulsgruppen, wobei die Pulse einer Pulsgruppe im wesentlichen auf denselben Ort einfallen. Dies gewährleistet, däss der Hauptpuls einer Pulsgruppe tatsächlich in das durch einen Vorpuls derselben Pulsgruppe modifizierte Materialvolumen treffen kann.

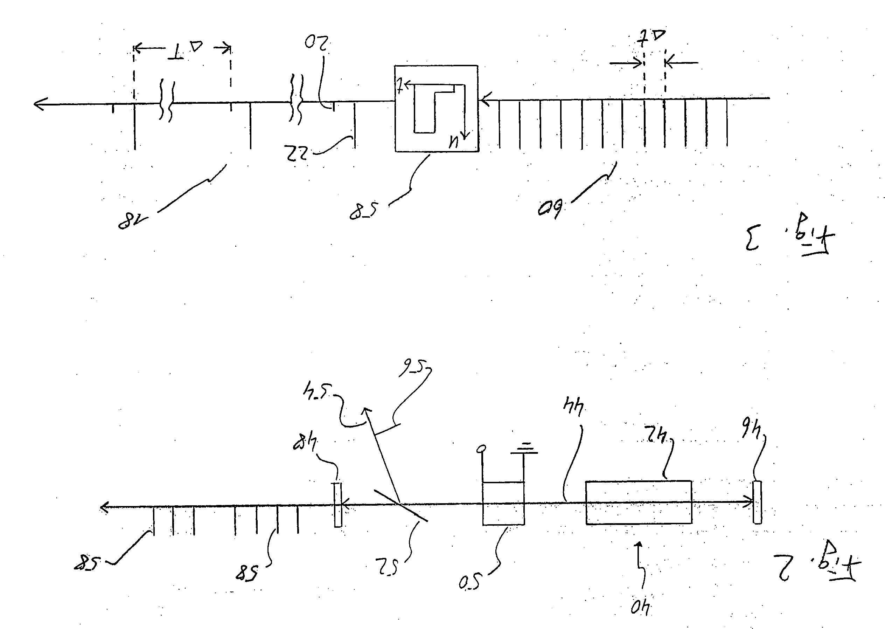

Es wird nun auf Figur 2 verwiesen. Diese Figur zeigt eine beispielhafte Konfiguration, die es gestattet, aus den von einem Laserresonator erzeugten Pulsen einzelne mittels eines elektro-optischen Schalters auszukoppeln. Der Laserresonator ist in Figur 2 allgemein mit 40 bezeichnet. Er weist in an sich bekannter Weise ein Lasermedium 42 auf, zu dessen beiden Seiten (bezogen auf die mit 44 bezeichnete Resonatorlängsachse) ein hochreflektierender Endspiegel 46 sowie ein teildurchlässiger Spiegel 48 angeordnet sind. Innerhalb des zwischen den beiden Spiegeln 46, 48 begrenzten Resonators sind ferner ein elektro-optischer Schalter 50, z.B. eine Pockels-Zelle, sowie eine beispielsweise von einem Dünnschichtpolarisator gebildete Polarisationsweiche 52 angeordnet. Mittels eines an den elektro-optischen Schalter 50 angelegten Spannungspulses kann die Polarisation eines im Resonator umlaufenden fs-Pulses gedreht werden, und zwar so, dass der polarisationsgedrehte Puls an der Polarisationsweiche 52 reflektiert und entlang einer Auskoppelrichtung 54 aus dem Resonator ausgekoppelt wird. Beispielhaft ist bei 56 ein solcher ausgekoppelter Einzelpuls gezeigt. Zu den Zeiten, zu denen der elektro-optische Schalter spannungsfrei ist, erfahren die im Resonator umlaufenden Pulse keine Polarisationsdrehung, weswegen sie durch die Polarisationsweiche 52 hindurchgehen und an dem teildurchlässigen Spiegel 48 ausgekoppelt werden. Solche entlang der Hauptresonatorachse 44 ausgekoppelten Pulse sind bei 58 beispielhaft angedeutet. Man erkennt, dass die von den Pulsen 48 gebildete Pulsfolge an der Stelle eine Lücke aufweist, wo der polarisationsgedrehte Puls 56 von der Polarisationsweiche 52 ausgekoppelt wurde.

Mit der Anordnung nach Figur 2 lässt sich somit durch Steuerung der an den elektro-optischen Schalter 50 angelegten Spannung ein einzelner Puls aus der von dem Laseroszillator 40 erzeugten Pulsfolge auskoppeln. Nimmt man eine Schaltgeschwindigkeit des elektro-optischen Schalters 50 im Bereich von etwa 1ns an, so ist diese Schaltgeschwindigkeit ausreichend schnell, um bei einer Pulswiederholrate des Oszillators 40 von etwa 10ns einzelne Pulse gezielt selektieren zu können.

Figur 2 stellt selbstverständlich nur eine von mehreren denkbaren Konfigurationen dar, um den Pulszug 18 der Figur 1 zu bilden. Es versteht sich, dass in Abwandlung der Beispielkonfiguration der Figur 2 zunächst alle in dem Oszillator umlaufenden Pulse als kontinuierliche Pulsfolge ausgekoppelt werden können und erst anschließend einzelne der Pulse mittels eines außerhalb des Oszillators angeordneten elektro-optischen Schalters ausgekoppelt werden. Dies entspricht im wesentlichen der Konfiguration gemäß Figur 1, wo der Pulsselektor 16 außerhalb des Oszillators 12 gezeigt ist.

Figur 3 verdeutlicht, wie durch Variation der an eine Pockels-Zelle 58 oder einen anderen elektro-optischen Schalter angelegten Spannung U der Pulszug 18 gemäß Figur 1 aus einer kontinuierlichen Pulsfolge 60 gebildet werden kann. In Figur 3 bezeichnet Δt den Zeitabstand zwischen aufeinanderfolgenden Pulsen der Folge 60, also die umgekehrte Pulswiederholrate, während ΔT den Zeitabstand zwischen aufeinanderfolgenden Pulsgruppen des Pulszugs 18 bezeichnet. Wie in dem in das Blockschaltsymbol für die Pockels-Zelle 58 eingezeichneten U(t)-Kennliniendiagramm (t bezeichnet die Zeit) gezeigt, kann mit einem stufenförmigen Verlauf der angelegten Spannung U eine Pulsgruppe mit einem energieschwächeren Vorpuls und einem energiestärkeren Hauptpuls gebildet werden. Gelangt während der niederen Spannungsstufe ein Puls der Folge 60 in die Pockels-Zelle 58, so wird dieser Puls als ein Vorpuls 20 selektiert, dessen Intensität dem Wert der Spannung U während der niederen Spannungsstufe entspricht. Gelangt dagegen ein Puls der Folge 60 während der höheren Spannungsstufe in die Zelle 58, so wird dieser Puls als ein Hauptpuls 22 durchgelassen, wobei vorzugsweise im wesentlichen keine Intensitätsabschwächung durch die Zelle 58 bewirkt wird. Zu allen übrigen Zeiten ist die an die Pockels-Zelle 58 angelegte Spannung Null. Dementsprechend wird während dieser Zeiten keiner der Pulse der Folge 60 durch die Zelle 58 transmittiert.

Es versteht sich, dass bei mehr als zwei Pulsen pro Pulsgruppe eine entsprechend modifizierte Spannungskennlinie zum Einsatz kommen kann. Insbesondere kann eine drei- oder mehrstufige Kennlinie verwendet werden, wenn nicht nur zwei Intensitätsstufen innerhalb einer Pulsgruppe vorkommen sollen, sondern drei oder mehr.

Die Pockels-Zelle 58 der Figur 3 kann in dem Impulsselektor 16 der Figur 1 implementiert sein oder den elektro-optischen Schalter 50 der Figur 2 bilden.

## Patentansprüche

1. Verfahren zur photodisruptiven Laserbearbeitung eines toten Materials, wobei bei diesem Verfahren ein Zug (18') von Laserstrahlungspulsen mit einer Pulsdauer im Femtosekundenbereich in Richtung auf das Material abgegeben wird, wobei der Pulszug eine Vielzahl aufeinanderfolgender Pulsgruppen umfasst, wobei jede Pulsgruppe mindestens zwei Laserstrahlungspulse (20', 22') umfasst, wobei bei dem Verfahren ferner die Laserstrahlungspulse einer Pulsgruppe im wesentlichen auf denselben Bearbeitungsort des Materials gerichtet werden, jedoch die Laserstrahlungspulse aufeinanderfolgender Pulsgruppen auf im wesentlichen verschiedene Bearbeitungsorte des Materials gerichtet werden, **dadurch gekennzeichnet, dass** der zeitliche Abstand zweier aufeinanderfolgender Laserstrahlungspulse einer Pulsgruppe im Nanosekundenbereich liegt.

2. Verfahren nach Anspruch 1, **dadurch gekennzeichnet, dass** zwei aufeinanderfolgende Laserstrahlungspulse (20', 22') einer Pulsgruppe mit einem zeitlichen Abstand abgegeben werden, der weniger als 100 ns, vorzugsweise weniger als 50 ns, höchstvorzugsweise weniger als 20 ns beträgt, beispielsweise im einstelligen Nanosekundenbereich bis hin zu höchstens etwa 10 ns.

3. Verfahren nach Anspruch 1 oder 2 **dadurch gekennzeichnet, dass** von zwei aufeinanderfolgenden Laserstrahlungspulsen einer Pulsgruppe ein vorlaufender Puls (20') geringere Energie oder/und geringere maximale Intensität als der nachfolgende Puls (22') besitzt.

4. Verfahren nach Anspruch 3, **dadurch gekennzeichnet, dass** die Energie oder/und die maximale Intensität des vorlaufenden Pulses (20') höchstens die Hälfte, vorzugsweise höchstens ein Viertel, höchstvorzugsweise höchstens ein Zehntel der Energie bzw. der maximalen Intensität des nachfolgenden Pulses (22') beträgt, beispielsweise näherungsweise ein Zwanzigstel.

5. Verfahren nach einem der Ansprüche 1 bis 4, **dadurch gekennzeichnet, dass** jede Pulsgruppe insgesamt zwei Laserstrahlungspulse (20', 22') umfasst.

6. Verfahren nach einem der Ansprüche 1 bis 5, **dadurch gekennzeichnet, dass** zwei aufeinanderfolgende Pulsgruppen mit einem zeitlichen Abstand abgegeben werden, der wenigstens eine Größenordnung, vorzugsweise mehrere Größenordnungen größer als der zeitliche Abstand aufeinanderfolgender Laserstrahlungspulse einer Pulsgruppe ist.

7. Verfahren nach einem der Ansprüche 1 bis 6 oder dem Oberbegriff des Anspruchs 9, **dadurch gekennzeichnet, dass** für jeden Laserstrahlungspuls (20', 22') des abgegebenen Pulszugs durch elektro-optisches Schalten jeweils ein Puls aus einer von einem Laseroszillator (12) erzeugten Folge von in gleichen Zeitabständen aufeinanderfolgenden Pulsen selektiert wird.

8. Verfahren nach Anspruch 7, **dadurch gekennzeichnet, dass** die Intensität der selektierten Pulse so moduliert wird, dass wenigstens ein Teil der selektierten Pulse unterschiedliche Pulsintensität hat.
